(19) Europäisches Patentamt

European Patent Office

Office européen des brevets

(11) **EP 0 914 826 A1**

(12) **EUROPEAN PATENT APPLICATION**
published in accordance with Art. 158(3) EPC

(43) Date of publication:
**12.05.1999 Bulletin 1999/19**

(51) Int. Cl.$^6$: **A61K 38/05**

(21) Application number: **97928460.1**

(22) Date of filing: **25.06.1997**

(86) International application number:
**PCT/JP97/02193**

(87) International publication number:
**WO 98/02177 (22.01.1998 Gazette 1998/03)**

(84) Designated Contracting States:
**AT BE CH DE DK ES FI FR GB GR IE IT LI LU MC NL PT SE**

(30) Priority: **17.07.1996 JP 187294/96**

(71) Applicants:
  • **ZERIA PHARMACEUTICAL CO., LTD.**
    **Chuo-ku, Tokyo 103 (JP)**
  • **Hamari Chemicals Co., Ltd.**
    **Osaka-shi, Osaka 533 (JP)**

(72) Inventors:
  • **YONETA, Tomoyuki.**
    **Zeria Pha. Co., Ltd. C. Res. Lab**
    **Konan-machi, Osato-gun Saitama 360-01-1, (JP)**
  • **YOSHIDA, Akihiko,**
    **Zeria Pharm.Co., Ltd. C .Lab.**
    **Konan-machi, Osato-gun, Saitama 360-01 (JP)**
  • **TAKAMI, Tokiro**
    **Suita-shi Osaka 565 (JP)**
  • **NISHIMURA, Yasuhiro**
    **Takarazuka-shi Hyogo 665 (JP)**

(74) Representative:
    **Hartz, Nikolai F., Dr. et al**
    **Wächtershäuser & Hartz,**
    **Patentanwälte,**
    **Tal 29**
    **80331 München (DE)**

(54) **WOUND HEALING ACCELERATORS**

(57)    Accelerators for healing wounds involving loss of skin tissue which contain as the active ingredient L-carnosine zinc salt or L-carnosine-zinc complex. These drugs are excellent in the effects of accelerating the healing of wounds such as open wound, combustio, congelatio, and decubitus.

EP 0 914 826 A1

**Description**

TECHNICAL FIELD

The present invention relates to a drug which accelerates healing of wounds involving loss of skin tissue, such as open wound, combustio, congelatio, and decubitus.

BACKGROUND ART

Wounds are generally classified into two groups according to whether or not they involve loss of skin tissue. The mechanism of healing has been clarified to also depend on whether the wound involves loss of skin tissue. In an incised wound, which is a wound that does not involve loss of skin tissue, the mechanism of healing centers on reproduction of connective tissue — primary healing. In contrast, examples of wounds involving loss of skin tissue include open wound, combustio, congelatio, and decubitus. A primary healing mechanism of these wounds consists of replacement of missing portions with granulation tissue and covering the granulation tissue with regenerating epidermis extending from the circumferential portions surrounding the injuries — secondary healing. Thus, the healing mechanism of a wound largely depends on whether it involves loss of skin tissue. This means that a medicine exhibiting an effect of accelerating healing by primary healing does not always exhibit the same effect for healing by secondary healing.

Conventionally, solcoseryl, L-carnosine, zinc, etc. have been utilized for the treatment of wounds. These substances have effects of accelerating healing by primary healing, but do not have satisfactory effect of accelerating healing by secondary healing.

Wounds involving loss of skin tissue are generally rather serious and require much time for healing. Among these type of wounds, decubitus are necrotic changes resulting from local ischemic circulatory disorders caused by continuous application of pressure to the skin, and thus are clinically serious conditions. However, so far no satisfactory therapeutic drug therefor has been known.

Therefore, there has been demanded development of drugs which accelerate growth of granulation tissue and regeneration of epidermis in wounds involving loss of skin tissue.

DISCLOSURE OF THE INVENTION

In view of the foregoing, the present inventors have conducted careful studies, and have found that L-carnosine zinc salt or L-carnosine-zinc complex has an accelerating effect on the growth of granulation tissue and regeneration of epidermis in wounds involving loss of skin tissue, leading to completion of the present invention.

Accordingly, an object of the present invention is to provide an accelerator for healing of wounds involving loss of skin tissue, which contains L-carnosine zinc salt or L-carnosine-zinc complex as the active ingredient.

Another object of the present invention is to provide a method for accelerating healing of wounds involving loss of skin tissue by administration of an effective amount of L-carnosine zinc salt or L-carnosine-zinc complex.

Yet another object of the present invention is to provide use of L-carnosine zinc salt or L-carnosine-zinc complex in manufacture of an accelerator for healing of wounds involving loss of skin tissue.

BEST MODE FOR CARRYING OUT THE INVENTION

L-Carnosine and zinc which serve as constituents of the L-carnosine zinc salt and L-carnosine-zinc complex (hereinafter abbreviated as L-carnosine zinc) are individually known to induce the aforementioned primary healing in wounds. Particularly, zinc has been incorporated in an ointment as zinc oxide and has long been used in this form as a wound healing agent.

The L-carnosine zinc of the present invention exhibits preventive and therapeutic effect against hepatitis, peptic ulcers, pancreatitis, and inflammatory bowel diseases. It exhibits more excellent effect than zinc sulfate in tests using a model of incised wound in the skin, and the mechanism that provides this effect has been clarified to be attributed to regeneration of connective tissue (Folia Pharmacologica Japonica, Vol. 100, pages 165-172, 1992). However, whether L-carnosine zinc has an effect of accelerating growth of granulation tissue has not yet been known.

L-carnosine zinc salt and L-carnosine-zinc complex assume two types of forms: amorphous and crystalline. Both types cannot be distinguished in terms of effect. Whether the L-carnosine zinc salt or the L-carnosine-zinc complex is amorphous or crystalline can be confirmed, for example, through observation under an electron microscope, infrared absorption spectrometry, or X-ray diffractometry.

L-Carnosine zinc may be prepared by causing L-carnosine to react with a zinc salt and an alkali metal compound in water or in an organic solvent.

Specifically, crystalline L-carnosine zinc can be obtained by reacting 0.8-1.2 mol zinc salt and 1.6-2.4 mol alkali metal compound based on 1 mol L-carnosine in a hydrous or anhydrous organic polar solvent at room temperature or by the application of heat (Japanese Patent Application Laid-Open No.. 64-42471). Examples of organic polar solvents include alcohols such as methanol, ethanol, propanol; acetonitrile; dimethylsulfoxide; N,N-dimethylformamide; tetrahydrofuran; and acetone. Organic polar solvents containing up to 50% water may also be added. The zinc salt which may be used is that of an inorganic acid or organic acid. Examples of zinc salts of inorganic acid include zinc halide, zinc sulfate, zinc nitrate, and zinc perchlorate, and examples of zinc salts of organic acid include zinc salts of carboxylic acid, such as zinc acetate, and acetyl acetone zinc. So long as the reaction proceeds, any zinc salt may be used. Examples of alkali metal compounds include lithium hydroxide, potassium hydroxide, sodium hydroxide, potassium alcoholate, and sodium alcoholate.

Amorphous L-cartnosinate or L-carnosine-zinc complex may be prepared in a manner similar to that described above except that water is used instead of the hydrous or anhydrous organic polar solvent.

The thus-obtained L-carnosine zinc may be combined with pharmaceutically acceptable additives, to thereby formulate topical agents for external use, such as ointments, pastes, cataplasms, powders for external use, sprays, and suppositories. Particularly, ointments and pastes are preferred.

In the drug for external application of the present invention, different pharmaceutically acceptable additives may be used in accordance with the type of dosage forms of the drug. Examples of additives include hydrocarbons such as Vaseline, liquid paraffin, and hydrocarbon gel (also called plastibase); animal and vegetable oils such as medium-chain fatty acid triglycerides, safflower oil, lard, hard fat, and cacao fat; higher fatty acid alcohols, fatty acids, and their esters, such as cetanol, stearyl alcohol, stearic acid, and isopropyl parmitate; water-soluble bases such as macrogol (polyethylene glycol), 1,3-butylene glycol, glycerol, gelatin, sucrose, and sugaralcohol; emulsifiers such as glycerin fatty acid esters, polyoxyl stearate, and polyoxyethylene hydrogenated castor oil; tackifiers such as acrylic acid esters, and sodium alginate; propellants such as liquefied petroleum gas and carbon dioxide gas; and preservatives such as paraoxybenzoic acid esters. The drug for external use of the present invention may be produced by use of these ingredients in a customary method.

In use, the drugs for external use of the present invention are applied to the local wound site according to a customary method.

EXAMPLES

The present invention will now be described by way of a referential example, test examples, and an example. However, the present invention is not limited thereto.

Referential Example:

《Production of crystalline L-carnosine-zinc complex》

Sodium hydroxide (3.51 g) was dissolved in methanol (100 ml), and L-carnosine (9.96 g) was added to the resultant solution to obtain a homogeneous solution. A solution of zinc acetate dihydrate (9.67 g) in methanol (145 ml) was added dropwise to the homogeneous solution with stirring for 30 minutes. White precipitates gradually settled. After completion of addition, the solution was stirred for two hours, allowed to stand overnight, filtered, and then subjected to washing with water (140 ml). The washed material was air-dried at 80°C for 5 hours, to thereby yield white powdery crystals (12.4 g).

IR(KBr)cm$^{-1}$: 3292, 1626, 1563, 1389, 1262, 1230, 1117, 1060, 1030, 999, 982, 883, 787

| Elementary analysis (as $C_9H_{12}N_4O_3Zn$): | | | | |
|---|---|---|---|---|
| | C | H | N | Zn |
| Calculated (%) | 37.33 | 4.18 | 19.35 | 22.53 |
| Found (%) | 37.07 | 4.27 | 19.06 | 22.50 |

| X-ray analysis pattern | |
|---|---|
| Interplanar Spacing (d; Å) | Relative intensity (%; $I/I_0$) |
| 11.87 | 26 |
| 7.54 | 28 |
| 5.97 | 43 |
| 5.55 | 27 |
| 5.26 | 30 |
| 4.52 | 51 |
| 3.96 | 100 |
| 3.56 | 19 |
| 3.24 | 29 |
| 2.97 | 19 |
| 2.79 | 17 |
| 2.68 | 22 |
| 2.58 | 21 |
| 2.38 | 25 |
| 2.10 | 13 |
| 1.97 | 14 |
| 1.88 | 15 |
| 1.84 | 14 |
| 1.69 | 10 |
| 1.57 | 9 |
| 1.55 | 9 |
| 1.53 | 10 |

Test Example 1: Wound Healing Acceleration Effect

Sprague-Dawley male rats (body weight: about 200 g) were shaved on the back under anesthesia with pentobarbital-Na, and were disinfected with tincture of iodine applied on the back. Two round wounds were symmetrically prepared on the back by use of a round leather-cutting punch (inner diameter: 1 cm). From the day the wounds were produced, hydrocortisone (50 mg/kg) was subcutaneously administered once daily so as to delay healing of wounds. Ointments (200 mg each per wound) having the following compositions were applied once daily. After 8 days the areas of the wounded portions were measured, and were compared with those of the control group (to which no ointment was applied), and effect of accelerating healing of wounds was determined from the equation (1) described below.

(Composition)

| • Crystalline L-carnosine-zinc complex ointment | |
|---|---|
| Crystalline L-carnosine-zinc complex | 20% |
| Plastibase | 80% |
| | Total 100 |

| • L-carnosine ointment | |
|---|---|
| L-carnosine | 15.5% |
| Plastibase | 84.5% |
| | Total 100 |

| • Zinc oxide ointment | |
|---|---|
| Zinc oxide | 5.6% |
| Plastibase | 94.4% |
| | Total 100 |

(Manufacturing Method)

Each substance was sieved through a #60-mesh sieve for removal of large particles, and then the substance and plastibase at proportions indicated above were added to a planetary mixer (kneader) so that the total weight of the ingredients for homogenization became 1,200 g. Aliquots (100 g each) of the resultant product were charged into tubes to obtain ointment preparations.

$$\text{Healing acceleration rate (\%)} = [\{(\text{Wound area of the control group}) - (\text{wound area of the test-drug application group})\} / (\text{Wound area of the control group})] \times 100 \quad (1)$$

Table 1

| Test drug | N | Wound area (mm$^2$) | Healing rate (%) |
|---|---|---|---|
| Control | 14 | 62.4±4.1 | |
| Base (plastibase) | 16 | 43.7±3.3** | 30.0 |
| Crystalline L-carnosine-zinc complex ointment | 14 | 31.8±2.5**,# | 49.0 |
| Zinc oxide ointment | 16 | 35.3±2.0** | 43.4 |
| L-Carnosine ointment | 16 | 47.4±2.7** | 24.0 |

Significance against the control group; **) $p<0.01$,
Significance against the base group; #) $p<0.05$,

As is apparent from Table 1, crystalline L-carnosine-zinc complex ointment of the present invention significantly reduced the wound area as compared to the base agent group, exhibiting an excellent wound healing acceleration effect.

Test Example 2: Cell Proliferation Acceleration Effect

Human umbilical vein endothelial cells (produced by Kurabo Industries Ltd.) were plated to a 96-well plate and incubated for 5 days. After washing of the endothelial cells twice with an MEM medium, the test-substance-containing MEM medium was added thereto, and the cells were incubated for 21 hours. 5-Bromo-2'-deoxy-uridine (BrdU) was added and incubation was continued for a further 3 hours. The culture medium was discarded, and the cells were fixed for 30 minutes in a fixing solution, followed by measurement of the amount of BrdU by ELISA. The results are shown in Table 2.

Table 2

| Test drug | Concentration (M) | BrdU(OD450nm), Mean±S.E. |
|---|---|---|
| Control | | 0.224±0.035 |
| Crystalline L-carnosine-zinc complex | $1\times10^{-10}$ | 0.347±0.028* |
| | $1\times10^{-9}$ | 0.722±0.024** |
| L-Carnosine | $1\times10^{-10}$ | 0.261±0.034 |
| | $1\times10^{-9}$ | 0.232±0.022 |
| | $1\times10^{-8}$ | 0.209±0.017 |
| Zinc sulfate | $1\times10^{-10}$ | 0.235±0.041 |
| | $1\times10^{-9}$ | 0.335±0.043 |
| | $1\times10^{-8}$ | 0.392±0.033** |
| n=7, | | |

Significance against the control group; *) $p<0.05$, **) $p<0.01$.

Table 2 shows that administration of crystalline L-carnosine-zinc complex has an excellent wound healing acceleration effect as compared with administration of L-carnosine alone or zinc sulfate alone.

Test Example 3: Antibacterial Test

Test samples were prepared by dilution with dimethylsulfoxide (dilution series: 40,000, 20,000, 10,000, 5,000, 2,500, 1,250 mg/l). Each of the test samples (0.5 ml) was added to sensitivity test medium-N (manufactured by Nissui Pharmaceutical Co., Ltd..) (9.5 ml), followed by mixing and pouring onto a petri dish to obtain solid plates (concentrations of the test substance in agar medium: 2,000, 1,000, 500, 250, 125, 62.5 mg/l).

*S. aureus* was used as the inoculation bacterium, and culturing was performed in sensitivity measuring bouillon (by Nissui Pharmaceutical Co., Ltd.) at 37°C for 20 hours to obtain a suspension of about $10^6$ CFU/ml. By use of a platinum loop, each test bacterium suspension was applied, in a manner of drawing lines, to agar plates which had been mixed with the test substance, and the plates were cultured at 37 ± 1°C for 18-20 hours. The concentration at which no growth was observed, which is considered to be the minimal inhibitory concentration (MIC), was obtained. The results are shown in Table 3.

Table 3

| | (mg/l) |
|---|---|
| Test substance | Test bacterium |
| | *S. aureus* |
| Crystalline L-carnosine-zinc complex | 2000 |
| L-carnosine | >2000 |
| Zinc oxide | >2000 |
| Mixture of carnosine + Zinc oxide | >2000 |

As is apparent from Table 3, crystalline L-carnosine-zinc complex has an effect of inhibiting bacterial infections which delay the healing of wounds.

Formulation Example 1: Oil-based ointment

| Crystalline L-carnosine-zinc complex | 2 g |
|---|---|
| Liquid paraffin | 20 g |
| White vaseline | Amount to make total weight 100 g |

A 2% ointment was prepared by a customary method based on the above formulation.

Formulation Example 2: Water-soluble ointment

| Crystalline L-carnosine-zinc complex | 2 g |
|---|---|
| Polyethylene glycol 4000 | 40 g |
| Polyethylene glycol 400 | Amount to make total weight 100 g |

A 2% ointment was prepared by a customary method based on the above formulation.

Formulation Example 3: Cream for external use

| Crystalline L-carnosine-zinc complex | 2 g |
|---|---|
| Liquid paraffin | 10 g |
| White vaselin | 10 g |
| Stearyl alcohol | 5 g |
| 1,3-Butylene glycol | 5 g |
| Glycerin | 10 g |
| Polyoxyl 40 stearate | 3 g |
| Xanthan gum | 0.1 g |
| Butyl p-hydroxybenzoate | 0.2 g |
| Purified water | Amount to make total weight 100 g |

A 2% cream for external use was prepared by a customary method based on the above formulation.

Formulation Example 4: Suppositories

| Crystalline L-carnosine-zinc complex | 2 g |
|---|---|
| Hard fat | Amount to make total weight 100 g |

A 2% suppository was prepared by a customary method based on the above formulation.

INDUSTRIAL APPLICABILITY

The present invention provides excellent acceleration effects in healing of wounds involving loss of skin tissue, such as open wound, combustio, congelatio, and decubitus.

**Claims**

1.    An accelerator for healing of wounds involving loss of skin tissue, which contains as an active ingredient L-carnosine zinc salt or L-carnosine-zinc complex.

2.    An accelerator for healing of wounds involving loss of skin tissue according to Claim 1, wherein the accelerator is a drug for external use.

3.    A method for accelerating healing of wounds involving loss of skin tissue comprising administration of an effective amount of L-carnosine zinc salt or L-carnosine-zinc complex.

4.    A method according to Claim 3, wherein the administration is effected to wound sites.

5.    Use of L-carnosine zinc salt or L-carnosine-zinc complex for producing an accelerator for healing of wounds involving loss of skin tissue.

6.    Use according to Claim 5, wherein the accelerator is a medicine for external use.

# INTERNATIONAL SEARCH REPORT

| International application No. |
|---|
| PCT/JP97/02193 |

### A. CLASSIFICATION OF SUBJECT MATTER

Int. Cl$^6$  A61K38/05

According to International Patent Classification (IPC) or to both national classification and IPC

### B. FIELDS SEARCHED

Minimum documentation searched (classification system followed by classification symbols)

Int. Cl$^6$  A61K38/00-38/58, A61K33/30

Documentation searched other than minimum documentation to the extent that such documents are included in the fields searched

Electronic data base consulted during the international search (name of data base and, where practicable, search terms used)

CAS ONLINE (CARNOSIN?, ZINC?, TOPICAL?, WOUND?, DERMAL?, SKIN?, FROSTBIT?, BURN?, SCAD?)

### C. DOCUMENTS CONSIDERED TO BE RELEVANT

| Category* | Citation of document, with indication, where appropriate, of the relevant passages | Relevant to claim No. |
|---|---|---|
| X | Nihon Yakurigaku Zasshi, (Folia pharmacol. japon.), Vol. 100, No. 2 (1992) P. 165-172 | 1, 2, 5, 6 |
| Y | JP, 53-127835, A (Kineshiro Nagai), November 8, 1978 (08. 11. 78), Full descriptions; particularly claim; page 3, lower left column, lines 11, 12 (Family: none) | 1, 2, 5, 6 |
| Y | JP, 62-36314, A (Ed Gaistorich Zeene AG. fur Chemisch Industry) | 1, 2, 5, 6 |

☐ Further documents are listed in the continuation of Box C.   ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| September 17, 1997 (17. 09. 97) | September 30, 1997 (30. 09. 97) |

| Name and mailing address of the ISA/ | Authorized officer |
|---|---|
| Japanese Patent Office | |
| Facsimile No. | Telephone No. |

Form PCT/ISA/210 (second sheet) (July 1992)

INTERNATIONAL SEARCH REPORT

International application No.

PCT/JP97/02193

---

**Box I**   Observations where certain claims were found unsearchable (Continuation of item 1 of first sheet)

This international search report has not been established in respect of certain claims under Article 17(2)(a) for the following reasons:

1. [X] Claims Nos.: 3, 4
   because they relate to subject matter not required to be searched by this Authority, namely:
   
   Claims 3 and 4 pertain to methods for treatment of the human or animal body by therapy and thus relate to a subject matter which this International Searching Authority is not required, under the provisions of Article 17(2)(a)(i) of the PCT and Rule 39.1(iv) of the Regulations under the PCT, to search.

2. [ ] Claims Nos.:
   because they relate to parts of the international application that do not comply with the prescribed requirements to such an extent that no meaningful international search can be carried out, specifically:

3. [ ] Claims Nos.:
   because they are dependent claims and are not drafted in accordance with the second and third sentences of Rule 6.4(a).

---

**Box II**   Observations where unity of invention is lacking (Continuation of item 2 of first sheet)

This International Searching Authority found multiple inventions in this international application, as follows:

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

Remark on Protest   [ ]   The additional search fees were accompanied by the applicant's protest.
                    [ ]   No protest accompanied the payment of additional search fees.

Form PCT/ISA/210 (continuation of first sheet (1)) (July 1992)